# EUROPÄISCHE PATENTANMELDUNG

(11) **EP 1 359 532 A2**
(43) Veröffentlichungstag der Anmeldung: **05.11.2003**
(21) Anmeldenummer: 03008958.5
(22) Anmeldetag: 16.04.2003
(51) Int. Cl.: G06F 19/00

(54) **Verfahren zum Bedienen eines medizinischen Gerätes oder Systems sowie medizinisches Gerät oder System**

(30) Priorität: 29.04.2002 DE 10219097
(71) Anmelder: Siemens Audiologische Technik GmbH, 91058 Erlangen (DE)
(72) Erfinder: Aschoff, Stefan, 91088 Bubenreuth (DE); Bindner, Jörg, 91085 Weisendorf (DE); Weidner, Roland, 96199 Zapfendorf (DE)
(74) Vertreter: Berg, Peter, Dipl.-Ing.

(57) **Zusammenfassung**

Die Bedienung eines medizinischen Gerätes oder Systems (1) soll vereinfacht und verbessert werden. Hierzu schlägt die Erfindung vor, Daten bezüglich möglicher Bearbeitungs- oder Bediensequenzen in einer Datenbank (4) abzulegen. Aus dem Vergleich dieser Daten mit den von dem Benutzer (3) durchgeführten Bearbeitungs- oder Bedienschritten werden Hinweise auf weitere sinnvolle Bearbeitungs- oder Bedienschritte generiert sowie Einstellungen und Funktionen des medizinischen Gerätes oder Systems (1) automatisch ausgeführt.

## Beschreibung

Verfahren zum Bedienen eines medizinischen Gerätes oder Systems sowie medizinisches Gerät oder System

Die Erfindung betrifft ein Verfahren zum Bedienen eines medizinischen Gerätes oder Systems durch eine Person durch Ausführen einer Folge von Bearbeitungs- oder Bedienschritten. Ferner betrifft die Erfindung ein medizinisches Gerät oder System mit einer Bedieneinrichtung zum Durchführen von Bearbeitungs- oder Bedienschritten an dem medizinischen Gerät oder System.

Softwaregesteuerte medizinische Geräte sowie Systeme in Verbindung mit einem softwaregesteuerten medizinischen Gerät zeichnen sich zunehmend dadurch aus, dass ihre Funktionalität und ihre Einsatzmöglichkeiten zunehmen, da viele technische Abläufe und Funktionen mittels Software verhältnismäßig einfach realisierbar sind. Damit steigt jedoch auch die Wahrscheinlichkeit, dass die komplexen Bedienmöglichkeiten für einen Benutzer nicht mehr transparent gemacht werden können, womit die Wahrscheinlichkeit von Fehlbedienungen der Geräte und der zugehörigen Software zunimmt. Des Weiteren ist es für Produktmanager und Produktentwickler nicht einfach, die Fehlerpotentiale abzugreifen.

Bei Neuentwicklungen medizinischer Geräte und Systeme wurden bisher aufwändige Studien, Diskussionsgruppen, Kundenbefragungen und Ähnliches durchgeführt, um die Fehlerpotentiale bei der Bedienung der Geräte und Systeme zu erkennen. Sobald eine Betriebssoftware der Geräte und Systeme im Markt ist, werden Schulungen durchgeführt, Informationsmaterial erstellt und gegebenenfalls Fragebögen verschickt und ausgewertet, um unter anderem die Fehlerpotentiale zu erkennen und darauf einzugehen.

Bei den medizinischen Geräten und Systemen im Zusammenhang mit der Erfindung handelt es sich insbesondere um komplexe medizinische Geräte und Systeme, wie beispielsweise Tomographiegeräte, Angiographiegeräte oder Hörhilfegeräte, die auch mit einem Programmiersystem verbunden sein können.

Der Begriff "Bedienen" ist im Zusammenhang mit der Erfindung im weitesten Sinne zu verstehen und umfasst insbesondere das Steuern, Programmieren, Einstellen, Warten, Anpassen und/oder Überwachen des medizinischen Gerätes oder Systems.

Aus der DE 199 33 524 A1 ist ein Verfahren zur Eingabe von Daten in ein System bekannt. Um die Eingabe von Daten für Benutzer zu erleichtern, ist vorgesehen, dass auf eine Eingabe eines Benutzers hin ein oder mehrere zu dieser Eingabe möglichst genau passende Begriffe als erkannte Begriffe ermittelt werden, dass zu jedem dieser erkannten Begriffe ein Zuverlässigkeitswert festgestellt wird und dass die einer Eingabe zugeordneten Begriffe unter Berücksichtigung ihrer Zuverlässigkeitswerte behandelt werden.

Der Erfindung liegt die Aufgabe zugrunde, die Bedienung medizinischer Geräte und Systeme zu vereinfachen und zu verbessern.

Diese Aufgabe wird bei einem Verfahren zum Bedienen eines medizinischen Gerätes durch eine Person durch Ausführen einer Folge von Bearbeitungs- oder Bedienschritten dadurch gelöst, dass ein Vergleich der ausgeführten Folge von Bearbeitungsoder Bedienschritten mit in einer Datenbank abgelegten Daten möglicher Bearbeitungs- oder Bediensequenzen und eine Optimierung der Bedienung in Abhängigkeit des Vergleichsergebnisses erfolgen.

Ferner wird die Aufgabe bei einem medizinischen Gerät oder System mit einer Bedieneinrichtung zum Durchführen von Bearbeitungs- oder Bedienschritten an dem medizinischen Gerät oder System gelöst durch eine mit dem medizinischen Gerät oder System verbundene Datenbank zum Speichern von Daten möglicher Bearbeitungs- oder Bediensequenzen des medizinischen Gerätes oder Systems, Vergleichsmittel zum Vergleich der Bearbeitungs- oder Bedienschritte mit den in der Datenbank gespeicherten Daten und Steuermittel zur Steuerung von Ausgabemitteln in Abhängigkeit des Vergleichsergebnisses.

Zur Optimierung der Bedienung sieht die Erfindung einen Vergleich einer ausgeführten Folge von Bearbeitungs- oder Bedienschritten mit in einer Datenbank abgelegten Daten bezüglich möglicher Bearbeitungs- oder Bediensequenzen vor. Aufgrund der ausgeführten Bearbeitungs- oder Bedienschritte können Hinweise über sinnvoll an die ausgeführten Bearbeitungs- oder Bedienschritte anschließende Bearbeitungs- oder Bedienschritte gegeben werden. Weiterhin ist es möglich, Warnhinweise zu geben, falls die ausgeführte Folge von Bearbeitungs- oder Bedienschritten nicht sinnvoll erscheint.

Zum Ausführen der Bearbeitungs- oder Bedienschritte weist das medizinische Gerät bzw. System eine Bedieneinrichtung auf. Diese kann Schalter, Tasten, Spracheingabe usw. umfassen. Ebenso steht auch zur Ausgabe der Bedienhinweise eine Vielzahl unterschiedlicher Mittel zur Verfügung. Insbesondere umfassen diese Mittel akustische Mittel, wie Signaltöne oder Sprachausgabe, und optische Mittel, wie Bildschirm oder Display.

Eine Ausführungsform der Erfindung sieht vor, dass bei einer erkannten Bearbeitungs- oder Bediensequenz Parametereinstellungen oder Funktionen des medizinischen Gerätes oder Systems automatisch ausgeführt werden.

Bei der Erfindung werden bestimmte, von einer Person ausgeführte Folgen von Bearbeitungs- oder Bedienschritten, sogenannte Bearbeitungs- oder Bediensequenzen oder "use cases", erkannt, z.B. durch Vergleich mit den in der Datenbank abgelegten Bearbeitungs- oder Bediensequenzen, und protokolliert. Vorteilhaft werden auch neue Folgen von Bearbeitungs- oder Bedienschritten, also Folgen, zu denen es keine entsprechenden in der Datenbank abgelegten Bearbeitungs- oder Bediensequenzen gibt, protokolliert. Insbesondere bei den zuletzt genannten Folgen von Bearbeitungs- oder Bedienschritten ist es erforderlich, eine Bewertung herbeizuführen, um diesen sinnvolle Bedienhinweise, beispielsweise sinnvoll im Anschluss ausführbare Bedienschritte, zuordnen zu können. Vorteilhaft erfolgt hierzu zunächst ein Vergleich der neuen Folge von Bearbeitungs- oder Bedienschritten mit in einer weiteren Datenbank, der Masterdatenbank, abgelegter Daten möglicher Bearbeitungs- oder Bediensequenzen.

Die Datenbank und die Masterdatenbank sind vorzugsweise über ein Computer-Netzwerk, z.B. das Internet, miteinander verbunden. Dadurch können in der Masterdatenbank Daten möglicher Bearbeitungs- oder Bediensequenzen einer Vielzahl gleichartiger medizinischer Geräte und Systeme zentral gespeichert sein und dort entsprechend gepflegt und bewertet werden. Ausgehend von der Masterdatenbank kann dann die dem medizinischen Gerät oder System direkt zugeordnete Datenbank über das Computer-Netzwerk aktualisiert und erweitert werden. Bei einer von der Person durchgeführten Folge von Bearbeitungs- oder Bedienschritten, zu der keine Daten in der Datenbank vorhanden sind, erfolgt zunächst ein Vergleich mit den Daten der in der Masterdatenbank abgelegten Bearbeitungs- oder Bediensequenzen. Ist eine mit der Folge von Bearbeitungs- oder Bedienschritten übereinstimmende und bereits bewertete Bearbeitungs- oder Bediensequenz vorhanden, so werden diese Daten in die dem medizinischen Gerät oder System lokal zugeordnete Datenbank übertragen, so dass bei erneutem Ausführen der Folge von Bearbeitungs- oder Bedienschritten Hinweise zur Bedienung des medizinischen Gerätes oder Systems erfolgen bzw. Einstellungen und Funktionen automatisch ausgeführt werden können.

Die Datenübertragung zwischen den Datenbanken kann manuell ausgelöst werden. Vorzugsweise erfolgt sie jedoch automatisch, beispielsweise in Verbindung mit einer zeitlichen Steuerung.

Kann einer Folge von Bearbeitungs- oder Bedienschritten weder eine Bearbeitungs- oder Bediensequenz der dem medizinischen Gerät oder System lokal zugeordneten Datenbank noch eine Bearbeitungs- oder Bediensequenz der Masterdatenbank zugeordnet werden, so sieht die Erfindung vorteilhaft die Einbringung von Expertenwissen vor. Hierzu wird einem Experten die von dem Benutzer ausgeführte und protokollierte Folge von Bearbeitungs- oder Bedienschritten über das Computer-Netzwerk zugänglich gemacht. Der Experte analysiert die Folge von Bearbeitungs- oder Bedienschritten und bewertet bzw. optimiert sie gegebenenfalls. Vorteilhaft werden einzelnen Bearbeitungs- oder Bedienschritten der so entstandenen bewerteten Bearbeitungs- oder Bediensequenz Bedienhinweise und gegebenenfalls automatisch ausführbare Parametereinstellungen und Funktionen zugeordnet. So kann bei einer Folge von Bearbeitungs- oder Bedienschritten jeweils ein Hinweis auf mögliche weitere sinnvolle Bearbeitungs- oder Bedienschritte erfolgen. Die Daten bezüglich der so gebildeten Bearbeitungs- oder Bediensequenz werden dann vorteilhaft in der Masterdatenbank abgelegt. Über diese kann dann eine Vielzahl über das Netzwerk mit der Masterdatenbank verbundener Datenbanken medizinischer Geräte und Systeme aktualisiert und erweitert werden. Die neu bewertete Bearbeitungs- oder Bediensequenz steht dann auch für diese Geräte und Systeme zur Verfügung.

Selbstverständlich können durch den Experten in Verbindung mit der Masterdatenbank auch Daten einer Bearbeitungs- oder Bediensequenz in einer einem medizinischen Gerät oder System lokal zugeordneten Datenbank aktualisiert bzw. verändert werden.

Ein Vorteil der Erfindung liegt in der Vermeidung von Fehlbedienungen. Kann durch eine bestimmte Folge von Bearbeitungsoder Bedienschritten eines Benutzers ein beabsichtigtes Vorhaben des Benutzers, also eine beabsichtigte Bearbeitungsoder Bediensequenz, eindeutig erkannt werden, so können einem ausgeführten Bedienschritt sinnvoll nachfolgende Bedienschritte angezeigt werden, Einstellungen von Parametern und Funktionen automatisch ausgeführt werden oder auch bestimmte Funktionen des Systems gesperrt werden. Fehlbedienungen lassen sich so verhindern.

Weiterhin trägt die Erfindung dazu bei, durch Abgabe von Bedienhinweisen für einen Benutzer oder die automatische Ausführung von Funktionen den Durchsatz mit dem medizinischen Gerät oder System zu steigern.

Ein weiterer Vorteil der Erfindung liegt in dem neuartigen Servicekonzept, bei dem einem Experten, der Bearbeitungsoder Bediensequenzen des medizinischen Gerätes oder Systems bearbeitet, viel Routinearbeit abgenommen wird, indem er nur noch neue Folgen von Bearbeitungs- oder Bedienschritten zur Bearbeitung und Bewertung vorgelegt bekommt. Dadurch kann er sich auf die herausfordernden und schwierigen Sonderfälle konzentrieren, in denen er wirklich gebraucht wird. Bereits bearbeitete und in der Masterdatenbank abgelegte und bewertete Bearbeitungs- oder Bediensequenzen können dem Benutzer eines medizinischen Gerätes oder Systems automatisch übermittelt werden.

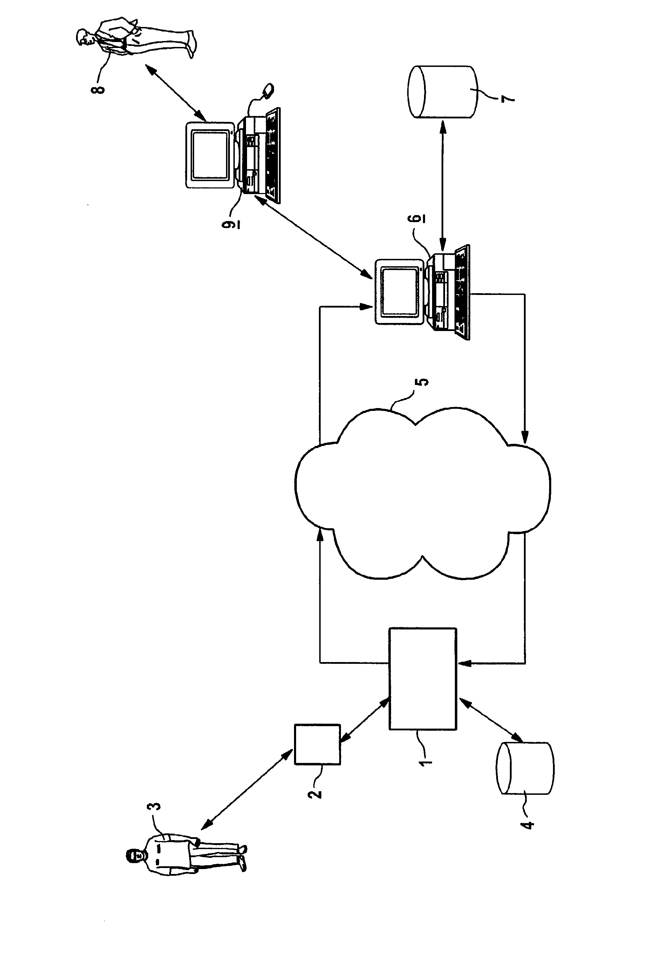

Weitere Einzelheiten der Erfindung werden nachfolgend anhand eines Ausführungsbeispieles näher erläutert. Dabei zeigt die Figur schematisch ein softwaregesteuertes medizinisches Gerät oder System 1, wie z.B. ein Hörhilfegerät in Verbindung mit einem Programmiersystem oder ein Computertomographiegerät. Das medizinische Gerät bzw. System 1 verfügt über Ein-/ Ausgabemittel 2, mittels derer ein Benutzer 3 das medizinische Gerät oder System 1 bedienen kann. Die Ein-/Ausgabemittel 2 können z.B. eine Tastatur und einen Monitor umfassen. Der Benutzer 3 führt über die Ein-/Ausgabemittel 2 eine Folge von Bearbeitungs- oder Bedienschritten zur Bedienung des medizinischen Gerätes oder Systems 1 aus. Dabei wird unter dem Begriff "Bedienen" in Zusammenhang mit der Erfindung insbesondere auch das Steuern, Programmieren, Einstellen, Warten, Anpassen sowie Überwachen des medizinischen Gerätes oder Systems verstanden. Die von dem Benutzer 3 über die Ein/Ausgabemittel 2 ausgeführte Folge von Bearbeitungs- oder Bedienschritten wird durch eine dem medizinischen Gerät oder System 1 zugeordnete Betriebssoftware in Verbindung mit einem Prozessor (nicht dargestellt) überwacht. Diese Betriebssoftware versucht, die beabsichtigte Anwendung (use case) des Benutzers 3 zu erkennen und diese Anwendung zu optimieren. Zum Erkennen des Vorhabens des Benutzers 3 wird ein Vergleich der von dem Benutzer 3 ausgeführten Bearbeitungs- oder Bedienschritte mit Daten bzgl. in einer Datenbank 4 abgelegten Bearbeitungs- oder Bediensequenzen ausgeführt. Stimmt die ausgeführte Folge von Bearbeitungs- oder Bedienschritten mit einem Teil einer in der Datenbank 4 gespeicherten Bearbeitungs- oder Bediensequenz überein, so können dem Benutzer 3 über die Ein-/Ausgabemittel 2 Hinweise bezüglich sinnvoller weiterer Bearbeitungs- oder Bedienschritte gegeben werden. Diese Hinweise können mit der entsprechenden Bearbeitungsoder Bediensequenz verknüpft ebenfalls in der Datenbank 4 gespeichert sein und durch den Prozessor, die Betriebssoftware und entsprechende Ausgabemittel in von dem Benutzer 3 wahrnehmbare Bedienhinweise gewandelt werden.

Ferner ist es möglich, dass, wenn die Betriebssoftware eine von dem Benutzer 3 beabsichtigte Anwendung, also eine beabsichtigte Bearbeitungs- oder Bediensequenz, erkennt, Einstellungen und Funktionen des medizinischen Gerätes oder Systems 1 automatisch ausgeführt werden. Hierzu werden aufgrund der Daten, die diese Bearbeitungs- oder Bediensequenz betreffen und die in der Datenbank 4 gespeichert sind, durch den Prozessor und die Betriebssoftware entsprechende Befehle generiert und ausgeführt. Dies kann dazu beitragen, insbesondere bei komplexeren Anwendungen des medizinischen Gerätes oder Systems 1, die Bedienung erheblich zu erleichtern und zu vereinfachen.

Kann der von dem Benutzer 3 ausgeführten Folge von Bearbeitungs- oder Bedienschritten nicht eindeutig eine in der Datenbank 4 gespeicherte Bearbeitungs- oder Bediensequenz zugeordnet werden, so stellt das medizinische Gerät oder System 1 über das Internet 5 eine Verbindung zu einem Rechner 6 her, der seinerseits mit einer Masterdatenbank 7 verbunden ist. Sowohl das medizinische Gerät oder System 1 als auch der Rechner 6 verfügen hierzu über die (bekannten) erforderlichen Verbindungsmittel. Die Kontaktaufnahme mit der Rechnereinheit 6 kann automatisch oder manuell veranlasst sein. Insbesondere ist es zweckmäßig, dass eine durch den Benutzer 3 durchgeführte Folge von Bearbeitungs- oder Bedienschritten, die keine Entsprechung einer in der Datenbank 4 gespeicherten Bearbeitungs- oder Bediensequenz findet, zunächst protokolliert wird, bevor ein Kontakt mit der Rechnereinheit 6 hergestellt wird. Die Daten bezüglich dieser neuen Bearbeitungsoder Bediensequenz werden dann mit Daten der in der Masterdatenbank 7 gespeicherten Bearbeitungs- oder Bediensequenzen verglichen. Ist die von dem Benutzer 3 ausgeführte Bearbeitungs- oder Bediensequenz in der Masterdatenbank 7 enthalten, so erfolgt eine Aktualisierung der Datenbank 4 mit den Daten bezüglich der neuen Bearbeitungs- oder Bediensequenz aus der Masterdatenbank 7.

Findet eine von dem Benutzer 3 ausgeführte Bearbeitungs- oder Bediensequenz weder in der Datenbank 4 noch in der Masterdatenbank 7 eine Entsprechung, so erfolgt vorteilhaft eine Bearbeitung und Bewertung dieser Bearbeitungs- oder Bediensequenz durch einen Experten 8. Dieser verwendet hierfür eine Rechnereinheit 9, auf der eine Expertensoftware zur Analyse der von dem Benutzer 3 ausgeführten Bearbeitungs- oder Bedienschritte ausgeführt wird. Der Experte 8 bearbeitet, überprüft und bewertet die ausgeführte Bearbeitungs- oder Bediensequenz und stellt insbesondere fest, ob diese
- a): vernünftig ist,
- b): unvernünftig ist, da es beispielsweise eine vernünftigere, weil effizientere oder effektivere Entsprechung gibt, oder
- c): fehlerhaft ist, da etwa die Bedienung in der angegebenen Weise nicht beabsichtigt ist.

Weiterhin werden die einzelnen Schritte der Bearbeitungsoder Bediensequenz mit zusätzlichen Informationen versehen, die dem Benutzer 3 während der Durchführung der Bearbeitungsoder Bediensequenz Hinweise auf sinnvolle, einem bestimmten Bearbeitungs- oder Bedienschritt nachfolgende Schritte geben sowie eventuell Einstellungen und Funktionen des medizinischen Gerätes oder Systems 1 automatisch ausführen. Die Daten der so bearbeiteten, neuen Bearbeitungs- oder Bediensequenz werden dann in der Masterdatenbank 7 und gegebenenfalls in der Datenbank 4 abgelegt. Vorteilhaft wird die Verwendungshäufigkeit der mit einem medizinischen Gerät oder System 1 ausgeführten und in der Datenbank 4 gespeicherten Bearbeitungs- oder Bediensequenzen protokolliert.

Die Software zur Steuerung des medizinischen Gerätes oder Systems 1 kann nun im laufenden Betrieb auf die aktualisierten Daten bezüglich der Bearbeitungs- oder Bedienschritte zugreifen und bei Erkennen einer beabsichtigten und in der Datenbank 4 vorhandenen Bearbeitungs- oder Bediensequenz Hinweise zur Optimierung der Abläufe geben oder Einstellungen und Funktionen automatisch durchführen. Weiterhin kann die Software, falls entsprechend vorbereitet, Abläufe selbständig optimieren.

Die Erfindung zeichnet sich aus durch eine Kombination aus zusätzlicher Software-Funktionalität, Vernetzung der betroffenen Rechner und einer Dienstleistung, die zum einen aus einer Aktualisierung der betroffenen Datenbanken und zum anderen aus einer Analyse und Optimierung des Betriebes des medizinischen Gerätes besteht.

Konkret kann das medizinische Gerät oder System 1 als System mit einem Hörhilfegerät und einem damit verbundenen Programmiergerät, auf dem eine Anpass-Software ausgeführt wird, ausgeführt sein. Die Geräte werden zunehmend komplexer und die Benutzer (Hörgeräteakustiker) sind zunehmend überfordert, da sie einerseits know-how über die Anpassung der Hörgeräte gegenüber den Schwerhörigen beweisen wollen und müssen, indem sie möglichst alle Funktionen der Software und der Hörhilfegeräte kennen und dem Kunden zeigen können sollten, aber andererseits die Hörhilfegeräte unübersehbar viele Möglichkeiten bieten. So wird oftmals zwar eine akzeptable, nicht jedoch die optimale Einstellung eines Hörhilfegerätes erreicht. Hier kann die Erfindung helfen, indem die Anpass-Software zusammen mit einer Datenbank ausgeliefert wird, in der Daten bezüglich möglicher Bearbeitungs- oder Bediensequenzen der Software und des Hörhilfegerätes gespeichert sind. Die Datenbank 4 enthält nicht nur die Daten sinnvoller Bearbeitungs- oder Bediensequenzen, sondern auch Daten bezüglich nicht sinnvoller Bearbeitungs- oder Bediensequenzen, so dass auch diese erkannt und Alternativen aufgezeigt werden. Eine mögliche Bediensequenz könnte die folgenden Bedienschritte umfassen:
- a): Import einer Lautheitsskalierungscharakteristik,
- b): Auslesen des angeschlossenen Hörhilfegerätes am rechten Ohr und
- c): Durchführen einer Voreinstellung mit Wahl einer Erwachsenencharakteristik ohne Lautheitsausgleich und mit einer Lautheitsskalierung ohne Reserve.

Ein nicht sinnvoller Bedienschritt könnte dabei die direkte Eingabe von Lautheitsskalierungsdaten sein. Unternimmt der Benutzer 3 Anstrengungen, diesen Bedienschritt auszuführen, so werden ihm automatisch eine Warnung sowie sinnvolle Alternativen, beispielsweise durch ein akustisches Warnsignal sowie eine Textausgabe auf einem Monitor mit sinnvollen Bedienschritten, aufgezeigt. Auch kann der Klick in das Lautheitsdiagramm auch automatisch einen Algorithmus zum Import von Lautheitsdaten bewirken. Dies ist jedoch nur möglich, wenn entsprechende Befehle der Bearbeitungs- und Bediensequenz als Daten zugeordnet und in der Datenbank 4 abgelegt sind.

## Patentansprüche

1. Verfahren zum Bedienen eines medizinischen Gerätes oder Systems (1) durch eine Person (3) durch Ausführen einer Folge von Bearbeitungs- oder Bedienschritten, **dadurch gekennzeichnet, dass** ein Vergleich der ausgeführten Folge von Bearbeitungs- oder Bedienschritten mit in einer Datenbank (4) abgelegten Daten möglicher Bearbeitungsoder Bediensequenzen und eine Optimierung der Bedienung in Abhängigkeit des Vergleichsergebnisses erfolgen.

2. Verfahren nach Anspruch 1, **dadurch gekennzeichnet, dass** zur Optimierung von der Person (3) wahrnehmbare Bedienhinweise erzeugt werden.

3. Verfahren nach Anspruch 1 oder 2, **dadurch gekennzeichnet, dass** zur Optimierung Parametereinstellungen und Funktionen des medizinischen Gerätes (1) automatisch ausgeführt werden.

4. Verfahren nach einem der Ansprüche 1 bis 3, **dadurch gekennzeichnet, dass** eine Folge von Bearbeitungs- oder Bedienschritten protokolliert wird.

5. Verfahren nach einem der Ansprüche 1 bis 4, **dadurch gekennzeichnet, dass** eine Folge von Bearbeitungs- oder Bedienschritten bewertet wird.

6. Verfahren nach Anspruch 5, **dadurch gekennzeichnet, dass** zur Bewertung ein Vergleich mit in einer Masterdatenbank (7) abgelegten Daten möglicher Bearbeitungs- oder Bediensequenzen erfolgt.

7. Verfahren nach Anspruch 5 oder 6, **dadurch gekennzeichnet, dass** die Bewertung durch einen Experten (8) erfolgt.

8. Verfahren nach einem der Ansprüche 5 bis 7, **dadurch gekennzeichnet, dass** Daten bezüglich der Folge von Bearbeitungs- oder Bedienschritten an einen Adressaten (6) eines Computer-Netzwerkes (5) gesendet werden.

9. Verfahren nach Anspruch 8, **dadurch gekennzeichnet, dass** die Daten automatisch gesendet werden.

10. Verfahren nach Anspruch 8 oder 9, **dadurch gekennzeichnet, dass** das Computer-Netzwerk (5) das Internet ist.

11. Verfahren nach einem der Ansprüche 5 bis 10, **dadurch gekennzeichnet, dass** Daten bezüglich der bewerteten Folge von Bearbeitungs- oder Bedienschritten in einer Masterdatenbank (7) abgelegt werden.

12. Verfahren nach einem der Ansprüche 5 bis 10, **dadurch gekennzeichnet, dass** Daten bezüglich der bewerteten Folge von Bearbeitungs- oder Bedienschritten als Daten einer möglichen Bearbeitungs- oder Bediensequenz in der Datenbank (4) abgelegt werden.

13. Medizinisches Gerät oder System (1) mit einer Bedieneinrichtung (2) zum Durchführen von Bearbeitungs- oder Bedienschritten an dem medizinischen Gerät oder System (1), **gekennzeichnet durch** eine mit dem medizinischen Gerät oder System (1) verbundene Datenbank (4) zum Speichern von Daten möglicher Bearbeitungs- oder Bediensequenzen des medizinischen Gerätes oder Systems (1), Vergleichsmittel zum Vergleich der Bearbeitungs- oder Bedienschritte mit den in der Datenbank (4) gespeicherten Daten und Steuermittel zur Steuerung von Ausgabemitteln in Abhängigkeit des Vergleichsergebnisses.

14. Medizinisches Gerät oder System (1) nach Anspruch 13, **gekennzeichnet durch** Mittel zum automatischen Einstellen von Betriebsparametern des Medizinisches Gerätes oder Systems (1) in Abhängigkeit des Vergleichsergebnisses.

15. Medizinisches Gerät oder System (1) nach einem der Ansprüche 13 oder 14, , **gekennzeichnet durch** Speichermittel zum Speichern von Daten bezüglich der durchgeführten Bearbeitungs- oder Bedienschritte.

16. Medizinisches Gerät oder System (1) nach einem der Ansprüche 13 bis 15, **gekennzeichnet durch** Übertragungsmittel zum Senden von Daten bezüglich der durchgeführten Bearbeitungs- oder Bedienschritte an einen Adressaten (6) und zum Empfangen von Daten bezüglich möglicher Bearbeitungs- oder Bediensequenzen von dem Adressaten.

17. Medizinisches Gerät oder System (1) nach Anspruch 16, **dadurch gekennzeichnet, dass** die Übertragungsmittel das Internet umfassen.

18. Medizinisches Gerät oder System (1) nach einem der Ansprüche 13 bis 17, **gekennzeichnet durch** Mittel zum Bewerten möglicher Bearbeitungsoder Bedienschritte.

19. Medizinisches Gerät oder System (1) nach Anspruch 18, **gekennzeichnet durch** eine Masterdatenbank (8) zum Speichern von Daten bezüglich bewerteter Bearbeitungs- oder Bedienschritte als Daten einer möglichen Bearbeitungs- oder Bediensequenz.
